(19) Europäisches Patentamt
European Patent Office
Office européen des brevets

(11) Publication number: **0 104 018 A2**

(12) **EUROPEAN PATENT APPLICATION**

(21) Application number: 83305154.3

(22) Date of filing: 06.09.83

(51) Int. Cl.³: C 07 D 311/24
C 07 D 335/06, C 07 D 215/48
C 07 D 405/04, C 07 D 409/04
C 07 D 401/04, C 07 D 491/052
C 07 D 493/04, C 07 D 471/04
A 61 K 31/35, A 61 K 31/38
//(C07D491/052, 311/00, 249/00),
(C07D493/04, 335/00, 249/00),
(C07D471/04, 249/00, 221/00)

(30) Priority: 21.09.82 GB 8226899
25.09.82 GB 8227404
25.09.82 GB 8227405
25.09.82 GB 8227406
19.01.83 GB 8301366
21.01.83 GB 8301747
24.05.83 GB 8314286
10.06.83 GB 8315939
11.06.83 GB 8316010

(43) Date of publication of application:
28.03.84 Bulletin 84/13

(84) Designated Contracting States:
AT BE CH DE FR GB IT LI LU NL SE

(71) Applicant: FISONS plc
Fison House Princes Street
Ipswich Suffolk IP1 1QH(GB)

(72) Inventor: Hazard, Richard
14 Ridley Close
Cropston Leicestershire(GB)

(72) Inventor: Dicker, Ian Douglas
36 Main Street Normanton-on-Soar
Loughborough Leicestershire(GB)

(72) Inventor: Ingall, Anthony Howard
17 Sandringham Drive
Loughborough Leicestershire(GB)

(72) Inventor: Cox, David
60 Atherstone Road
Loughborough Leicestershire(GB)

(72) Inventor: Wilkinson, David John
12 Cleeve Mount
Loughborough Leicestershire(GB)

(74) Representative: Craig, Christopher Bradberry et al,
Fisons plc 12 Derby Road
Loughborough Leicestershire LE11 0BB(GB)

(54) **Benzopyran, benzothiapyran and quinoline compounds and pharmaceutical compositions containing them.**

(57) These are described compounds of formula I,

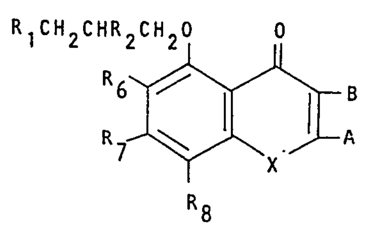

I

in which X is -O-, -S- or $-NR_3-$,
$R_3$ is hydrogen or alkyl C 1 to 6,
A is -COOH or 5- (1H-tetrazolyl), and B is hydrogen, or A and B together form a chain -N=N-NH-,
$R_1$ is hydrogen or hydroxy,
$R_2$ is hydroxy or esterified hydroxy,
$R_6$ is hydrogen or alkyl C 1 to 3,
$R_7$ is hydrogen or methyl, and
$R_8$ is alkyl C 1 to 4 optionally substituted by phenyl, provided that
when B is hydrogen, A is -COOH, X is -O-, $R_1$ is hydrogen, $R_2$ is hydroxy, $R_6$ and $R_7$ are both hydrogen then $R_8$ is not propyl, and pharmaceutically acceptable derivatives thereof.

There are also described methods for making the compounds and pharmaceutical, e.g. gastric acid secretion inhibiting, compositions containing them.

EP 0 104 018 A2

# BENZOPYRAN, BENZOTHIAPYRAN AND QUINOLINE COMPOUNDS AND PHARMACEUTICAL COMPOSITIONS CONTAINING THEM

This invention relates to new compounds, methods for their preparation and pharmaceutical compositions containing them.

A large number of monochromone-2-carboxylic acids are known as anti-allergic agents, and a small number of these compounds, in particular the compound described in British Patent Specification No 1,488,707, have been disclosed as having the ability to inhibit gastric acid secretion.

We have now found that a selected group of compounds have advantageous properties as disclosed herein.

According to the invention we provide compounds of formula I,

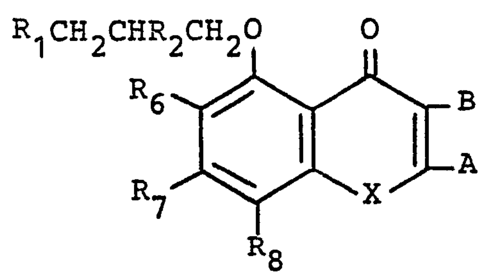

I

in which X is -O-, -S- or $-NR_3-$,

$R_3$ is hydrogen or alkyl C 1 to 6,

A is -COOH or 5-(1H-tetrazolyl), and B is hydrogen, or A and B together form a chain -N=N-NH-,

$R_1$ is hydrogen or hydroxy,

$R_2$ is hydroxy or esterified hydroxy,

$R_6$ is hydrogen or alkyl C 1 to 3,

$R_7$ is hydrogen or methyl, and

$R_8$ is alkyl C 1 to 4 optionally substituted by phenyl,

provided that

when B is hydrogen, A is -COOH, X is -O-, $R_1$ is hydrogen, $R_2$ is hydroxy, $R_6$ and $R_7$ are both hydrogen then $R_8$ is not propyl,

and pharmaceutically acceptable derivatives thereof.

According to our invention we also provide a process for the preparation of a compound of formula I, or a pharmaceutically acceptable derivative thereof, which comprises

a) producing a compound of formula I in which A and B together form a chain -N=N-NH- by removing a protecting group L from a compound of formula III,

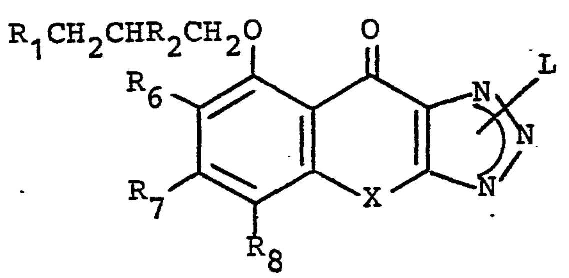

III

in which X, $R_1$, $R_2$, $R_6$, $R_7$ and $R_8$ are as defined above, and

L is a protecting group,

b) producing a compound of formula I in which B is hydrogen and A is 5-(1H-tetrazolyl), by reacting a compound of formula IV,

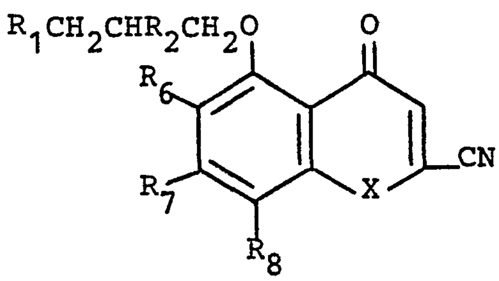

IV

in which X, $R_1$, $R_2$, $R_6$, $R_7$ and $R_8$ are as defined above,

with an azide, in solvent which is inert under the reaction conditions,

c) producing a compound of formula I in which $R_1$ or $R_2$ is hydroxy by selectively reducing a compound of formula V,

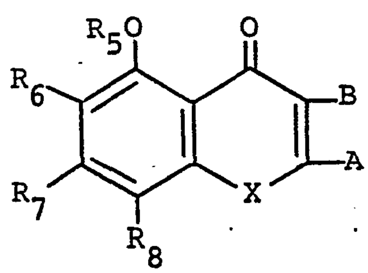

V

or a salt, ester or amide thereof,

in which X, A, B, $R_6$, $R_7$ and $R_8$ are as defined

above, and

$R_5$ represents a group $-CH_2CZCHZ$, where one Z represents carbonyl oxygen and the other represents $H_2$,

with a suitable selective carbonyl reducing agent,

d) producing a compound of formula I in which B is hydrogen and A is -COOH by hydrolysing a compound of formula VI,

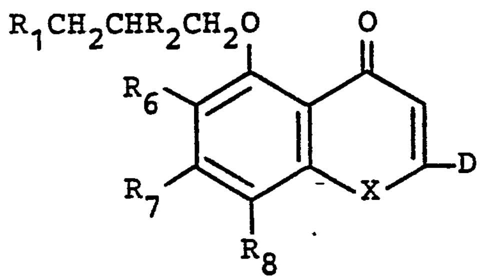

VI

in which D represents a group hydrolyseable to -COOH, and

X, $R_1$, $R_2$, $R_6$, $R_7$ and $R_8$ are as defined above,

e) producing a compound of formula I in which B is hydrogen and A is -COOH by cyclising a compound of formula VII,

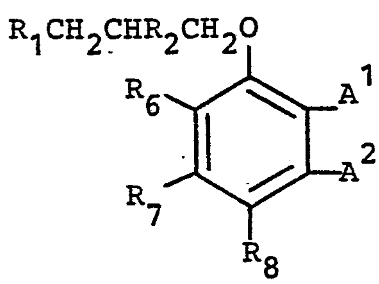

VII

in which $R_1$, $R_2$, $R_6$, $R_7$ and $R_8$ are as defined above,

$A^1$ and $A^2$ represent the pairs of groups

i) $-COCH_2COCOR"$ and -OM, or

ii) -H and -X-C(COR")=CH-COR"

X is as defined above,

R" represents -OM, or a group which is hydrolysable thereto, and

M represents hydrogen or a cation,

and if necessary or desired hydrolysing the group -COR", to a group -COOM,

f) reacting a compound of formula VIII,

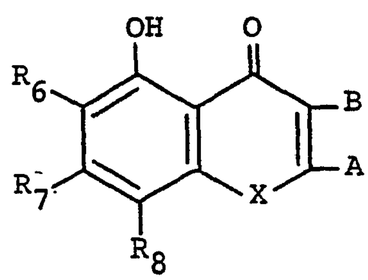

VIII

or a salt, ester or amide thereof,

in which X, A, B, $R_6$, $R_7$ and $R_8$ are as defined above,

with propylene oxide or a compound of formula IX,

$$R_1CH_2CHR_2CH_2G \qquad IX$$

in which G is a good leaving group,

g) producing a compound of formula I in which $R_1$ and $R_2$ are both hydroxy by

i) hydrolysing a compound of formula X,

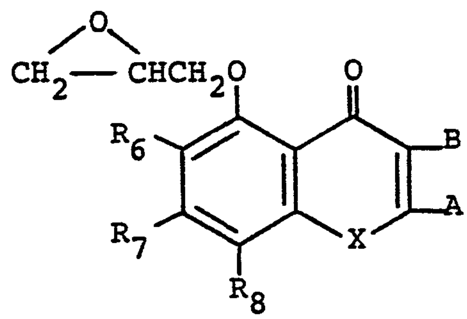

X

or a salt, ester or amide thereof,

in which X, A, B, $R_6$, $R_7$ and $R_8$ are as defined above, or

ii) subjecting a compound of formula XI,

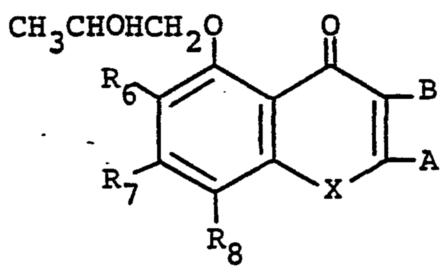

XI

in which X, A, B, $R_6$, $R_7$ and $R_8$ are as defined above,

or a salt, ester or amide thereof,

to mammalian metabolism, or

iii) reaction of a compound of formula XII,

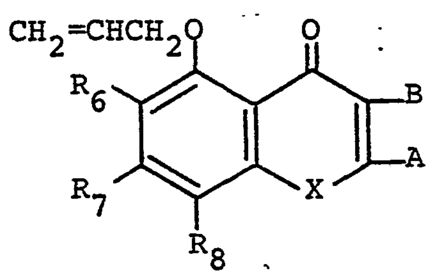

XII

or a salt, ester or amide thereof,

in which X, A, B, $R_6$, $R_7$ and $R_8$ are as defined above,

with osmium tetroxide, and hydrolysis of the resulting complex, or

h) producing a compound of formula I in which $R_2$ is esterified hydroxy, by reaction of a corresponding compound of formula I, or a salt, carboxylic ester or amide thereof, in which $R_2$ is hydroxy with an appropriate acid, or a derivative thereof,

and where desired or necessary converting the resulting compound of formula I to a pharmaceutically acceptable derivative thereof, or vice versa.

In process a) L may be an N-protecting group removable by acidolysis with a strong acid. Suitable examples of L include labile benzyl groups or triphenylmethyl. Examples of labile benzyl groups include benzyl substituted in the phenyl ring by one or more C 1-4 alkoxy groups, for example 4-methoxy-, 2,4-dimethoxy- or 2,4,6-trimethoxybenzyl. A particularly suitable example of L is 4-methoxybenzyl. Suitable acids include trifluoroacetic and methanesulphonic acids. The reaction may be carried out at a temperature of from 25 to 150°C, preferably 50 to 70°C.

L may also be an N-protecting group removable by

hydrogenolysis. Examples of such groups include 1-phenylalkyl groups, particularly benzyl. The hydrogenolysis may conveniently be carried out using conventional techniques known per se.

Suitable solvents which are inert under the reaction conditions of process b) include those in which both the reagents are soluble, e.g. N,N-dimethylformamide, dimethylsulphoxide, tetrahydrofuran, diethyl glycol and ethyl methyl glycol. The reaction is preferably carried out at a temperature of from about 20° to 130°C. The azide used in the reaction is preferably ammonium or an alkali metal azide, e.g. sodium or lithium azide. The reaction may, if desired, be carried out in the presence of an electron acceptor, e.g. aluminium chloride, boron trifluoride, ethyl sulphonic acid or benzene sulphonic acid. Alternatively the reaction may be carried out using hydrazoic acid under greater than atmospheric pressure. When an azide other than hydrazoic acid is used, e.g. sodium azide, the product of the reaction will be the corresponding tetrazole salt. This salt may readily be converted to the free acid by treatment with strong acid, e.g. hydrochloric acid.

Suitable selective carbonyl reducing agents in process c) include potassium or sodium borohydride, or aluminium isopropoxide. The reduction may be carried out

in a solvent which is inert under the reaction conditions, e.g. ethanol or dioxan, at a temperature of from about 5 to 75°C.

In process d), the group D may be, for example, an ester, preferably a lower alkyl ester, e.g. methyl or ethyl; an acid halide, e.g. chloride; an amide, e.g. -CONRxRy, where one or both of Rx and Ry represent hydrogen, or lower alkyl, e.g. methyl or ethyl; or a nitrile group, which may be hydrolysed to a -COOH group. The hydrolysis may be carried out using acid conditions, for example, using a hydrogen halide, e.g. hydrogen bromide or hydrogen chloride in acetic acid, or a mixture of aqueous dioxan and hydrochloric acid, or under mildly basic conditions, e.g. using sodium hydroxide, sodium carbonate or sodium bicarbonate, in a suitable solvent, inert to the reaction conditions, for example a lower alcohol, e.g. ethanol or methanol. The hydrolysis may be carried out at a temperature of from about 5 to 120°C, depending on the compounds used.

The cyclisation of process e) (i) may be carried out by heating, or under basic or neutral conditions. It is however preferred to carry out the cyclisation in the presence of an acid, e.g. hydrochloric acid, and in a solvent which is inert under the reaction conditions, e.g. ethanol. The reaction may be carried out at from about

$20^{o}$ to $150^{o}C$. The group -COR" is preferably an ester group, e.g. R" may be a lower alkoxy group. When M is a cation it may be, for example, an alkali metal cation.

The cyclisation of process e) (ii) may be carried out by treating the compound of formula VII with a cyclising agent, for example a dehydrating agent such as chlorosulphonic, polyphosphoric or sulphuric acid. The reaction is preferably carried out under anhydrous conditions and may be carried out at a temperature of from about $0^{o}$ to $100^{o}C$. Alternatively, cyclisation may be achieved by converting the free carboxy groups of the compound of formula VII to acyl halide groups and subjecting the resulting acyl halide to an intramolecular Friedel-Crafts reaction. As a further alternative, particularly when X is $-NR_3-$, the cyclisation may be carried out by heating, e.g. to a temperature of 200 to $300^{o}C$, with or without a solvent.

In process f) the good leaving group G may be, for example, an anion forming group, e.g. a chlorine, bromine or iodine atom or an alkane sulphonate group. The reaction is preferably carried out in a solvent which is inert under the reaction conditions, e.g. dimethylformamide, and in the presence of a strong base, e.g. sodium hydride. The reaction is preferably carried out at a temperature of from about 25 to $150^{o}C$.

The reaction involving propylene oxide may be carried out without a solvent or in a solvent which is inert under the reaction conditions, e.g., dimethylformamide. The reaction is preferably carried out at a temperature of from about 75° to 175°C in the presence of a base, e.g., sodium hydroxide or thallous ethoxide.

The hydrolysis of process g) (i) is preferably carried out in an aqueous medium, e.g. water, and under mildly basic or mildly acidic conditions. The reaction may conveniently be carried out at a temperature of from about 5 to 50°C, e.g. at room temperture.

Process g) (ii) may be carried out by administering the starting material to a suitable mammal or mammalian extract. The starting material may conveniently be administered in an aqueous medium, and the desired product may be isolated from the animal's urine, or from the extract, using conventional processes, e.g. high performance liquid chromatography.

The reaction of process g) (iii) may be carried out in the presence of hydrogen peroxide, and conveniently is carried out at about room temperature.

In process h) the esterification may be carried out by reacting the free OH group with a compound RCOG in which G is as described above for process f) and may also be -O-alkanoyl or trifluoroacetyl. The reaction is

preferably carried out in the presence of a non-nucleophilic proton acceptor, e.g. triethylamine or pyridine. Where the ester is with a dicarboxylic acid the starting material may be reacted with an appropriate anhydride. The reaction may be carried out in a solvent which is inert under the reaction conditions, e.g. a polar aprotic solvent such as 1,4-dioxan, dimethylformamide or N-methyl pyrrolidone, or a halogenated hydrocarbon, for example dichloromethane or dichloroethane. The reaction may be carried out at a temperature of from about 0 to 105°C.

In processes c), f), g) and h) when an ester starting material is used we prefer it to be a C 1 to 6, e.g. an ethyl, ester.

The starting materials for the above processes may be made by the processes described in the Examples or by processes analogous thereto or by processes analogous to those described above. Thus the compounds of formula III may be prepared by processes analogous to processes c), f), g) i), g) iii) or h) or by conventional processes known *per se*.

The compound of formula IV may be made from the corresponding free acid or from known compounds using conventional techniques known *per se*, e.g. conversion of the acid to an amide and dehydration of the amide to yield

the nitrile.

The compounds of formula V may be made by reacting a compound of formula VIII with a reagent $R_5G$ in which $R_5$ and G are as defined above, in the presence of a proton acceptor, e.g. potassium carbonate, in a solvent inert to the reaction conditions, e.g. acetone, at a temperature of from about 5 to 85°C.

The compounds of formula V in which $R_5$ represents $-CH_2COCH_3$ may also be made by reacting the corresponding compound of formula VIII or an ester, amide or salt thereof, with $HC \equiv CH_2G$, where G is as defined above (e.g. bromine) in the presence of a proton acceptor, e.g. potassium carbonate, in a suitable solvent, e.g. acetone, to give an intermediate in which $R_5$ represents $-CH_2-C{\equiv}CH$, followed by hydration of the acetylenic bond using water, in the presence of a suitable catalyst, e.g. mercuric chloride or mercuric acetate, in a suitable solvent, e.g. ethanol, containing an acid, e.g. p-toluenesulphonic acid.

The compounds of formula VI may be made by processes analogous to process c), e), f), g) i), g) iii) or h).

The compounds of formulae VII, VIII, IX, X, XI and XII are either known, or may be made from known compounds using conventional methods known *per se*.

The compounds of formula I and the intermediates

therefore may be isolated from their reaction mixtures using conventional techniques.

Pharmaceutically acceptable derivatives of the compounds of formula I include pharmaceutically acceptable salts, and, where A is -COOH, esters and amides of the 2-carboxylic acid group. Suitable salts include ammonium, alkali metal (e.g. sodium, potassium and lithium) and alkaline earth metal salts (e.g. calcium or magnesium), and salts with suitable organic bases, e.g. salts with hydroxylamine, lower alkyl-amines, e.g. hydroxy substituted alkylamines such as tris(hydroxymethyl) methylamine, or with simple monocyclic nitrogen heterocyclic compounds, e.g. piperidine or morpholine. Suitable esters include simple C 1 to 6 alkyl esters, e.g. the ethyl or butyl ester, esters derived from alcohols containing basic groups, e.g. di-lower alkyl amino substituted alkanols such as the 2-(diethylamino)-ethyl ester, and acyloxy alkyl esters, e.g. a lower acyloxy-lower alkyl ester such as the pivaloyloxymethyl ester, or a bis-ester derived from a di-hydroxy compound, e.g. a di(hydroxy-lower alkyl) ether, e.g. the bis-2-oxapropan-1,3-diyl ester. The esters may be made by conventional techniques, e.g. esterification, transesterification or reaction of the acid, or a salt thereof, with an appropriate compound containing a good

leaving group. The amides may be, for example, unsubstituted or mono- or di- C 1 to 6 alkyl amides and may be made by conventional techniques, e.g. reaction of an ester of the corresponding acid with ammonia or an appropriate amine.

The compounds of formula I and pharmaceutically acceptable derivatives thereof are useful because they possess pharmacological activity in animals; in particular they are useful because they inhibit the release and/or action of pharmacological mediators which result from the *in vivo* combination of certain types of antibody and specific antigen, e.g. the combination of reaginic antibody with specific antigen (see Example 27 of British Patent Specification No. 1,292,601). In man, both subjective and objective changes which result from the inhalation of specific antigen by sensitised subjects are inhibited by prior administration of the new compounds. Thus the new compounds are indicated for use in the treatment of asthma, e.g. allergic asthma. The new compounds are also indicated for use in the treatment of so-called 'intrinsic' asthma (in which no sensitivity to extrinsic antigen can be demonstrated). The new compounds may also be of value in the treatment of other conditions in which antigen-antibody reactions are responsible for disease, for example, hay fever; certain eye conditions, e.g.

trachoma; alimentary allergy, e.g. urticaria and atopic eczema; and gastrointestinal allergy, especially in children, e.g. milk allergy.

The new compounds may also be used for the prevention or inhibition of gastric acid secretion, and/or the treatment of conditions normally involving excess gastric acid secretion, e.g. peptic, duodenal, gastric, recurrent or stormal ulceration, dyspepsia, duodenitis, Zollinger-Ellison syndrome, reflux oesophagitis and the management of haemorrhage, e.g. from erosion or ulcers, in the upper gastrointestinal tract, especially when a major blood vessel is not involved. The compounds may also be used to reduce the chance of haemorrhage in patients with severe hepatic failure who are known to be at special risk. The above conditions may be treated whether or not they are associated with excess gastric acid secretion.

For the above mentioned uses the dosage administered will, of course, vary with the compound employed, the mode of administration and the treatment desired. However, in general, satisfactory results are obtained when the compounds are administered at a dosage of from 0.1 to 50 mg per kg of animal body weight in the test set out in Example 27 of the British Patent Specification No. 1,292,601. For man the indicated total daily dosage is in the range of from 1mg to 3,500mg preferably from 1mg to

3,000mg and more preferably from 1mg to 600mg, which may be administered in divided doses from 1 to 6 times a day or in sustained release form. Thus unit dosage forms suitable for administration (by inhalation or oesophageally) comprise from 0.17mg to 600mg, preferably 0.17mg to 500mg and more preferably from 0.17mg to 100mg of the compound preferably admixed with a solid or liquid pharmaceutically acceptable diluent, carrier or adjuvant.

The compounds of formula I, and pharmaceutically acceptable derivatives thereof, have the advantage that they are more efficacious in certain pharmacological models, are more readily absorbed, are longer acting, e.g. when inhaled, are more active, have less undesirable side effects or possess other advantageous properties than compounds of similar structure to the compounds of formula I.

According to the invention there is also provided a process for the production of a pharmaceutically acceptable salt of a compound of formula I, which comprises treating a compound of formula I or, where A is a -COOH group an ester thereof, or another salt thereof, with a compound containing an available pharmaceutically acceptable cation and capable of converting the compound of formula I or the salt or ester thereof, to a pharmaceutically acceptable salt of the acidic function

A/B.

Compounds capable of converting the compound of formula I, or the salt or ester thereof, to a pharmaceutically acceptable salt thereof include compounds, e.g. bases and ion exchange resins, containing pharmaceutically acceptable cations, e.g. sodium, potassium, calcium, ammonium and appropriate nitrogen containing organic cations.

In the compounds of formula I we prefer B to be hydrogen and A to be 5-(1H-tetrazolyl) or more preferably -COOH. $R_3$ may be hydrogen or alkyl C 1 to 3, e.g. ethyl. We particularly prefer X to be -S- or -O-. $R_1$ is preferably hydrogen. $R_2$ is preferably -OH or an ester thereof with a C 1 to 7 hindered mono- carboxylic acid, e.g. pivalic acid, or with an aryl C7 to 12 acid, e.g. benzoic acid or with a C3 to 7 dibasic acid, e.g. glutaric or succinic acid. $R_6$ may be, for example, hydrogen, ethyl or propyl. $R_7$ is preferably hydrogen. $R_8$ is preferably ethyl, propyl, butyl or benzyl.

According to our invention we also provide a pharmaceutical composition comprising (preferably less than 80%, and more preferably less than 50% by weight) of a compound of formula I, or a pharmaceutically acceptable derivative thereof, in combination with a pharmaceutically acceptable adjuvant, diluent or carrier. Examples of suitable

adjuvants, diluents or carriers are:for tablets, capsules and dragées; microcrystalline cellulose, calcium phosphate, diatomaceous earth, a sugar such as lactose, dextrose or mannitol, talc, stearic acid, starch, sodium bicarbonate and/or gelatin; for suppositories, natural or hardened oils or waxes; and for inhalation compositions, coarse lactose or aerosol propellants. The compound of formula I, or the pharmaceutically acceptable derivative thereof, preferably is in a form having a mass median diameter of from 0.01 to 10 microns. The compositions may also contain suitable preserving, stabilising and wetting agents, solubilizers, sweetening and colouring agents and flavourings. The compositions may, if desired, be formulated in sustained release form. We prefer compositions which are designed to be taken oesophageally and to release their contents in the gastrointestinal tract.

Certain of the compounds of formula I exist in optically active forms. The invention also provides the optical isomers of these compounds and mixtures, including racemic mixtures thereof. The compounds may be resolved into their optical isomers using conventional techniques.

The invention is illustrated, but in no way limited, by the following Examples in which temperatures are in $^{o}C$.

Example 1

5-[5-(2-Hydroxypropoxy)-4-oxo-8-propyl-4H-1-benzopyran-2-yl] 1H-tetrazole, sodium salt

A stirred mixture of 5-(2-hydroxypropoxy)-4-oxo-8-propyl-4H-1-benzopyran-2-carbonitrile,(9.1g), ammonium chloride, (2.05g), and sodium azide, (2.55g), in dry dimethylformamide, (100ml), was heated on a steam bath for 3 hours, poured into 3M HCl and extracted into methylene chloride. The extracts were washed well with water, dried over $MgSO_4$, filtered and evaporated. The residue was dissolved in sodium bicarbonate solution and washed by shaking with ethyl acetate. A solid was precipitated from the aqueous layer. This solid was filtered off, washed with ethyl acetate and with cold water, then dried, leaving the required salt as a pale fawn powder, (4.93g).

| Elemental Analysis | | C | H | N |
|---|---|---|---|---|
| | Found | 47.8% | 5.32% | 13.97% |
| $C_{16}H_{17}N_4NaO_4$ 12.17% $H_2O$ | Requires | 47.87% | 5.56% | 13.96% |

Example 2

1-Ethyl-1,4-dihydro-5-(2-hydroxypropoxy)-4-oxo-8-propylquinoline-2-carboxylic acid

a) N-Ethyl-N-(5-methoxy-2-propylphenyl)acetamide

A solution of N-(5-methoxy-2-propylphenyl)acetamide (5.0g) in dry N,N-dimethylformamide (10ml) was added

dropwise to a stirred suspension of ether washed 50% sodium hydride in oil (1.28g) in dry N,N-dimethyl formamide under nitrogen. The reaction mixture was stirred for 1h, cooled in ice and a solution of bromoethane (2ml) in dry N,N-dimethylformamide (10ml) added dropwise. The cooling bath was removed and the reaction stirred at room temperature for 2h, poured into ice and dilute hydrochloric acid and extracted with ether which was washed with water (4x50ml) and dried over magnesium sulphate. The solvent was evaporated to give 5.0g of the product as a pale yellow oil. The structure was confirmed by nmr and ms.

b) 3-Ethylamino-4-propylphenol

The product of step a) (17g) was heated under reflux in 70% w/w sulphuric acid (170ml) for 1h. The mixture was cooled, poured onto ice and basified to pH8 with ammonia solution (0.88 specific gravity). The product was extracted with ethyl acetate, washed with water and dried over magnesium sulphate. The solvent was evaporated to give 11.7g of the sub-title compound as a red oil. The structure was confirmed by nmr and ms.

c) Methyl 1-ethyl-1,4-dihydro-5-hydroxy-4-oxo-8-propylquinoline-2-carboxylate

A solution of the product of step b) (12.6g) and dimethyl acetylenedicarboxylate (9.5ml) in ethanol (50ml)

were heated under reflux for 1h. The reaction mixture was cooled, poured into water and extracted with ether which was washed with water and dried. The solvent was evaporated to give 21.5g of dimethyl N-ethyl-N-(5-hydroxy -2-propylphenyl)-2-aminobutene-1,4-dioate as a viscous oil. The structure was confirmed by nmr and ms. This product and polyphosphoric acid (100ml) were heated on a steambath with stirring for 10 min. The reaction mixture was poured onto a mixture of ethyl acetate and ice water, the organic layer was separated, washed with water and dried. The solvent was evaporated and the residue treated with ether and filtered. The filtrate was evaporated and eluted down a silica gel column using ether/petroleum ether (4:6) as eluant initially followed by ether/petroleum ether (4:1) to give 6.4g of the required sub-title product as an orange coloured oil. The structure was cofirmed by nmr and ms.

d) Methyl 1-ethyl-1,4-dihydro-4-oxo-8-propyl-5-(2-propynyloxy)quinoline-2-carboxylate

The hydroxyquinoline from step c) (6.37g) and anhydrous potassium carbonate (6.1g) were stirred in dry dimethylformamide (100ml) and treated with a solution of 80% propargyl bromide in toluene (7.5ml). The reaction mixture was stirred for 3 days, poured into water, and extracted with ether which was washed with water (3x100ml)

then brine (1x100ml) and dried. The solvent was evaporated to leave 6.9g of the sub-title product as a viscous oil which solidified on standing. The structure was confirmed by nmr and ms.

e) Methyl 1-ethyl-1,4-dihydro-4-oxo-5-(2-oxo-propyloxy)-8-propylquinoline-2-carboxylate

The propargyl ether from step d) (6.9g), p-toluenesulphonic acid (0.86g) in water (8ml), and mercuric acetate (0.48g) in ethanol (200ml) were heated and stirred under reflux for 0.75h. The reaction mixture was cooled, poured into water and extracted with ethyl acetate which was washed with water and dried. The solvent was evaporated, the residue treated with ether, filtered and the filtrate evaporated. The residue was triturated with petroleum ether (bp 30-40$^{o}$) to give 6.1g of the sub-title product as a yellow brown solid. A recrystallisation from cyclohexane gave the sub-title product as a yellow crystalline solid mp 74-76$^{o}$.

Elemental analysis: Found C, 66.06, H, 6.66, N, 4.05%

$C_{19}H_{23}NO_5$: Reqd C, 66.1, H, 6.67, N, 4.06%

f) Methyl 1-ethyl-1,4-dihydro-5-(2-hydroxypropyloxy)-4-oxo-8-propylquinoline-2-carboxylate.

A solution of the product of step e) (5.0g) in ethanol (350ml) and glacial acetic acid (1.76ml) was treated portionwise with sodium borohydride (1.04g) with

stirring. The reaction mixture was stirred for 0.25h, poured into water, and extracted with ether, which was washed with water and dried. The solvent was evaporated and the residue eluted down a silica gel column using ethyl acetate as eluant to give 3.7g of the sub-title product as a yellow brown oil. The structure was confirmed by nmr and ms.

g) Sodium 1-ethyl-1,4-dihydro-5-(2-hydroxypropyloxy)-4-oxo-8-propylquinoline-2-carboxylate

A solution of the product of step f) (3.53g) in methanol (50ml) was heated under reflux with the slow addition of N sodium hydroxide solution (10.16ml). The reaction mixture was evaporated until almost dry and treated with acetone to give a sticky solid. The supernatant was decanted and the residue dissolved in water and freeze dried to give 2.63g of the sub-title product as a light brown powder.

Elemental Analysis: Found C, 57.75, H, 6.39, N, 3.64%

$C_{18}H_{22}NaNO_5$ 4.85% water: Reqd C, 57.85, H, 5.9-6.43, N, 3.75%

Example 3

1,4-Dihydro-5-(2-hydroxypropoxy)-4-oxo-8-propylquinoline-2-carboxylic acid

a) N-(2-(1-Oxopropyl)-5-methoxyphenyl)acetamide

N-3-Methoxyphenylacetamide (82.5g) and propionyl

chloride (92.5g) were dissolved in dichloroethane (1 litre) stirred in an ice water bath and treated portionwise with anhydrous ferric chloride (162.5g) over 1h. The mixture was allowed to warm to room temperature and stirred at ambient for 2h, poured into water and stirred rapidly. The organic layer was separated, washed with water (3x100ml) and dried over sodium sulphate. The solvent was evaporated to give a brown oil which was repeatedly extracted with petroleum ether (bp 60-80°). The extracts were concentrated to give 34.5g of sub-title product as white needles mp 81-84°. The structure was confirmed by nmr and ms.

b) N-(5-methoxy-2-propylphenyl)acetamide

The product of step a) (2.2g) was dissolved in ethanol (80ml), concentrated hydrochloric acid (3 drops) added and the solution hydrogenated at atmospheric pressure in the presence of 10% palladium on charcoal (0.4g) until hydrogen uptake had ceased. The catalyst was filtered off and the filtrate evaporated to give a white solid which was recrystallised from cyclohexane to give 1.4g of the sub-title product as off white needles mp 112-113.5°C.

Elemental analysis: Found C, 69.55. H, 8.15, N, 6.81%

$C_{12}H_{17}NO_2$: Reqd C, 69.53, H, 8.27, N, 6.76%

c) 5-Methoxy-2-propylbenzeneamine

The amide of step b) (6.7g) was heated under reflux in methanol (80ml), concentrated hydrochloric acid (30ml) and water (30ml) for 2h. The reaction mixture was poured onto ice and basified with ammonia solution (0.88 specific gravity). The product was extracted into ether, washed with water and dried over magnesium sulphate. The solvent was evaporated to give 5.0g of the sub-title product as a pale yellow oil. The structure was confirmed by nmr and ms.

d) <u>Methyl 1,4-dihydro-5-methoxy-4-oxo-8-propylquinoline -2-carboxylate</u>

The amine of step c) (30.5g) and dimethyl acetylenedicarboxylate (25ml) were heated under reflux in ethanol (500ml) for 2h. The reaction mixture was evaporated to dryness to give 59.2g of dimethyl N-(5-methoxy-2-propylphenyl)-2-aminobutene-1,4-dioate as an oil. The oil (59.2g) was added to diphenyl ether (500ml) at reflux. The reaction mixture was heated under reflux for 10 min, cooled, poured into 60-80$^{\circ}$ petroleum ether and the precipitated product collected by filtration, washed well with petroleum ether and dried to give 35.5g of the product as a light brown solid. A recrystallisation from methanol gave a light brown crystalline sub-title product, mp 122-123$^{\circ}$C.

Elemental analysis: Found C, 65.39, H, 6.18, N, 5.07%

$C_{15}H_{17}NO_4$: Reqd C, 65.5, H, 6.18, N, 5.09%

e) Methyl 1,4-dihydro-5-hydroxy-4-oxo-8-propylquinoline -2-carboxylate

The methyl ether from step d) (8.0g) in dry dichloromethane (300ml) was treated with a one molar solution of boron trichloride in dry dichloromethane (88ml) at -70° with stirring. The reaction mixture was allowed to warm to room temperature during 1h, poured onto ice water, stirred for 1h and extracted with ether, which was washed with water and dried over magnesium sulphate. The solvent was evaporated and the residue heated under reflux in methanol (300ml) which had been saturated with hydrogen chloride gas for 24 hours. The reaction mixture was poured into water, and extracted with ether which was washed with water and dried. The solvent was evaporated and the residue dissolved in ethyl acetate, silica gel (100g) added and the solvent evaporated. The product was obtained by eluting down a silica gel column using ether as eluant to give 3.2g of the sub-title product as a yellow solid mp 90 - 92°.

Elemental analysis Found: C, 63.76, H, 5.71, N, 5.17%

$C_{14}H_{15}NO_4$ Reqd: C, 64.4, H, 5.75, N, 5.23%

f) Methyl 8-propyl-4,5-di(2-propynyloxy)quinoline-2-carboxylate

The hydroxyquinoline of step (e) (1.5g), anhydrous

potassium carbonate (3.16g) and 80% propargyl bromide in toluene (2.1ml) were stirred in dry dimethylformamide (100ml) for 4 days. The reaction mixture was poured into water, extracted with ethyl acetate, washed with water (4x) and dried over magnesium sulphate. The solvent was evaporated to give 1.6g of the sub-title product. A recrystallisation from methanol gave fluffy cream needles mp 136-137°.

Elemental analysis Found: C, 71.46, H, 5.81, N, 4.2%

$C_{20}H_{19}NO_4$ Reqd: C, 71.2, H, 5.64, N, 4.15%

g) <u>Methyl 4-methoxy-8-propyl-5-(2-propynyloxy)quinoline-2-carboxylate</u>

Methanol (500ml) was saturated with hydrogen chloride gas, treated with the product of step (f) (14g) and heated under reflux for 3 hours. The reaction mixture was concentrated to low volume, diluted with ether, washed with water, aqueous sodium bicarbonate solution then water again and dried over magnesium sulphate. The solvent was evaporated to give a mixture of the required product and methyl 1,4-dihydro-4-oxo-8-propyl-5-(2-propynyloxy) quinoline-2-carboxylate. This mixture was dissolved in dry dimethylformamide (150ml) treated with anhydrous potassium carbonate (6.0g) and methyl iodide (3.0ml), and stirred for 1 hour. The reaction mixture was poured into water, extracted with ethyl acetate, which was then washed

with water and dried. The solvent was evaporated and the residue recrystallised from cyclohexane to give 10.6g of the required sub-title product as a cream coloured solid mp 118-120°.

Elemental analysis Found: C, 68.98, H, 6.15, N, 4.42%

$C_{18}H_{19}NO_4$ Reqd: C, 69.0, H, 6.07, N, 4.47%

h) Methyl 4-methoxy-5-(2-oxopropyloxy)-8-propylquinoline -2-carboxylate

The propargyl ether of step (g) (4.75g, 0.0152mol), p-toluenesulphonic acid (0.6g) in water (7.1ml) and mercuric acetate (0.34g) in ethanol (100ml) were heated under reflux with stirring for 40 min.

The reaction mixture was cooled, poured into ethyl acetate and water, the organic layer separated, washed with water and dried over magnesium sulphate. The solvent was evaporated and the residue recrystallised from cyclohexane to give 2.8g of the sub-title product. The structure was confirmed by nmr and ms.

i) Methyl 1,4-dihydro-4-oxo-5-(2-oxopropyloxy)-8-propylquinoline-2-carboxylate

The product of step (h) (4.56g) in methanol (70ml), water (7ml) and conc. HCl (0.7ml) were heated under reflux for 7.5h. The reaction mixture was poured into dichloromethane, washed with water, aqueous sodium bicarbonate solution, then water again and dried over

magnesium sulphate. The solvent was evaporated, the residue triturated with ether, collected by filtration, washed with ether and dried to give 3.2g of the required sub-title product. A recrystallisation from methanol gave brown needles mp 168-169$^{o}$.

Elemental analysis: Found C, 64.16, H, 5.95, N, 4.26%

$C_{17}H_{19}NO_5$: Reqd C, 64.4, H, 6.0, N, 4.42%

j) Methyl 1,4-dihydro-5-(2-hydroxypropyloxy)-4-oxo-8-propylquinoline-2-carboxylate

The product of step i) (2.83g) was dissolved in dimethylformamide (60ml) with slight warming and treated with glacial acetic acid (1.1ml) followed by the slow addition of sodium borohydride (0.64g) with stirring. The reaction mixture was then stirred for 1h, poured into water, extracted with ethyl acetate, washed with water (4x) and dried. The solvent was evaporated and the residue eluted down a silica gel column using ethyl acetate/petroleum ether (4:1) as eluant to give 2.22g of the sub-title compound as a yellow gum which solidified on standing mp 78-81$^{o}$.

Elemental analysis: Found C, 63.81, H, 6.61, N, 4.23%

$C_{17}H_{21}NO_5$: Reqd C, 63.9, H, 6.58, N, 4.39%

k) Sodium 1,4-dihydro-5-(2-hydroxypropyloxy)-4-oxo-8-propylquinoline-2-carboxylate

The quinolone of step j) (1.906g) in methanol (40ml)

was heated under reflux with stirring with the slow addition of N sodium hydroxide solution (5.98ml). The reaction mixture was then heated and stirred under reflux for 0.5h, allowed to cool to room temperature and concentrated to dryness. The residue was dissolved in water, filtered and the filtrate freeze dried to give 1.745g of the title sodium salt.

Elemental analysis: Found C, 53.98, H, 5.26, N, 3.88

$C_{16}H_{18}NNaO_5$ 8.04% water: Reqd C, 53.98, H, 5.06-5.94, N, 3.94%

Gravimetric analysis showed 8.04% weight loss.

Example 4

5-(2-Hydroxypropoxy)-4-oxo-6,8-dipropyl-4H-1-benzopyran-2-carboxylic acid

(a) 2-Acetyl-4,6-dipropyl resorcinol

A mixture of 2-acetyl-4,6-diallyl resorcinol (30g) and 5% palladium on charcoal, (0.25g) in ethanol, (150ml) was shaken in 3 atmospheres pressure of hydrogen for 2 hours. Normal atmosphere was restored and catalyst was filtered off and destroyed. The filtrate was evaporated to leave sub-title material as a green solid 930g), m.p> 50°, which was satisfactory on nuclear magnetic resonance spectroscopy.

(b) 2-Hydroxy-6-(2-hydroxypropoxy)-3,5-dipropylacetophenone

A mixture of the product of step (a) (30g) with propylene oxide (15ml) and 5ml of a 40% solution of

benzyltri-methyl ammonium hydroxide in water, was dissolved in dioxan (250ml), sealed in a glass vessel and then heated in a steam-bath for 40 hours. The mixture was cooled and then solvent was evaporated off. The residue was dissolved in ether (150ml) and the solution was washed with water, then with 1% sodium hydroxide solution, again with water, dried ($MgSO_4$), filtered and evaporated to leave the sub-title material as a red oil, (20.5g). Nuclear magnetic resonance supported the structure proposed.

(c) 5-(2-Hydroxypropoxy)-4-oxo-6,8-dipropyl-4H-1benzopyran-2-carboxylic acid

To a stirred solution of sodium ethoxide, prepared by dissolving sodium (8g) in ethanol (250ml), was added a solution of the product of step (b) (20.5g) and diethyl oxalate, (25g) in ethanol (100ml). The mixture was stirred and kept under reflux for 5 hours, then it was poured into a stirred mixture of concentrated hydrochloric acid, (40ml), water (200ml) and chloroform (200ml). The chloroform layer was isolated and combined with a further chloroform wash of the aqueous layer. The total chloroform solution was washed with water, (2 x 200ml), dried ($MgSO_4$) filtered and evaporated. The residual oil was dissolved in ethanol, (100ml) and treated with conc. HCl, (2ml). This mixture was kept under reflux for 1 hour

after which the solvent was removed. The residue was treated with sodium bicarbonate, (8g) methanol, (50ml) and water (150ml) and this mixture was kept under reflux for 1 hour, evaporated and then extracted into water. The aqueous solution was washed with ether, filtered and then acidified with 5N hydrochloric acid. Precipitated material solidified upon standing and was subsequently dissolved in chloroform. The solution was washed with water and then evaporated to leave the title material, (8.6g), m.p. 135-7°.

Analysis:

Found: C, 65.8; H, 7.0

$C_{19}H_{24}O_6$ requires: C, 65.6; H, 6.94%

(d) Sodium 5-(2-hydroxypropoxy)-4-oxo-6,8-dipropyl-4H-1-benzopyran-2-carboxylate

The product of step (c) (8.2g) was dissolved in a solution of sodium bicarbonate, (2.1g) in water (40ml). The solution was filtered, evaporated to near dryness and then diluted with acetone. Crystallisation occurred. The crystals were filtered off, crushed, and dried *in vacuo* at 80° for 2 hours to leave the sub-title material as a white powder, (7.7g). Structural evidence was provided by nuclear magnetic resonance spectroscopy.

Analysis:

Found: C, 54.05; H, 6.6

$C_{19}H_{23}NaO_6.3H_2O$ requires: C, 53.8 ; H, 6.8%

Example 5

5-(2-Hydroxypropoxy)-7-methyl-4-oxo-8-propyl-4H-1-benzopyran-2-carboxylic acid

a) 1-(2-Hydroxy-4-methyl-6-[2-propenyloxy]phenyl) ethanone

1-(2,6-Dihydroxy-4-methylphenyl)ethanone (33.7g), $K_2CO_3$ (35g), allyl bromide (21ml) and dimethylformamide (400ml) were stirred together for 18 hours and poured into water. The precipitated sub-title compound was collected, dried and recrystallised from 60/80 petroleum ether. mp 84-86°.

b) 1-(2,6-Dihydroxy-4-methyl-3-(2-propenyl)phenyl) ethanone

The product of step a) (26g) was heated in tetralin (52ml) at 165-170° for 2 hours. After cooling the precipitated sub-title compound was collected, washed with petroleum ether and dried. mp 104-106°.

c) 1-(2-Hydroxy-6-[2-hydroxypropoxy]-4-methyl-3-[2-propenyl]phenyl)ethanone

The product of step b) (23.5g), propylene oxide (44ml), tetraethyl ammonium bromide (0.9g) and dioxan (30ml) were heated at 100° in a sealed container for 2.5 hours, then cooled and poured into water. Chloroform extraction, and washing with water followed by drying and

evaporation afforded the sub-title compound as an oil (30g) MS: $M^+$264 BP191.

d) 1-(2-Hydroxy-6-[2-hydroxypropoxy]-4-methyl-3-propyl phenyl) ethanone

The product of step c) (30g) was hydrogenated over 5% Pd/C (1g) at 1 atmosphere $H_2$ pressure in ethanol (200ml) until hydrogen uptake ceased. The catalyst was filtered off, and solvent was evaporated. The residue was triturated with petroleum ether, then recrystallised from 40/60 petroleum ether to afford the sub-title compound (15.6g) mp 76-77°.

e) Sodium 5-(2-hydroxypropoxy)-7-methyl-4-oxo-8-propyl-4H-1-benzopyran-2-carboxylate

Sodium hydride (2.8g) was added to a suspension of the product of step d) (15.6g) and diethyl oxalate (17.2g) in 60/80 petroleum ether (200ml). Gentle warming resulted in brisk effervescence and when this subsided the mixture was refluxed for 15 minutes. On cooling ethanol (65ml) and conc. HCl (16ml) were added. The mixture was heated on a steam bath for 10 minutes, then cooled, evaporated and extracted into chloroform. The organic solution was washed with water, dried and evaporated to afford the chromone ester as an oil (20g).

The oil (20g) was dissolved in methanol (50ml) together with sodium bicarbonate (7.6g) and water (90ml)

and refluxed for 2 hours. The solution was cooled, washed with chloroform, then acidified and extracted into $CHCl_3$. The extract was washed with water, dried and evaporated. The resulting acid was dissolved in a solution of sodium bicarbonate (50g) in water (100ml) with heating. The solution was filtered, cooled and diluted with acetone. The resulting precipitate was collected and dried to afford the title compound (11.2g) mp > 300°.

Example 6

6,8-Diethyl-5-(2-hydroxypropoxy)-4-oxo-4H-1-benzopyran-2-carboxylic acid

a) 1-[3,5-Diethyl-2-hydroxy-6-[2-hydroxypropoxy]phenyl) ethanone

1-(3,5-Diethyl-2,6-dihydroxy-phenyl)ethanone (16.9g), propylene oxide (15ml), benzyltrimethylammonium hydroxide (20 drops) and dioxan (30ml) were heated together in a sealed system at 100° for 21 hours. Volatile materials were then evaporated off and the residue was extracted into chloroform, which was washed with water, dried and evaporated to leave an orange oil (20.1g). NMR consistent with the desired structure.

b) 6,8-Diethyl-5-(2-hydroxypropoxy)-4-oxo-4H-1-benzopyran-2-carboxylic acid

To a solution of sodium (11.5g) in ethanol (450ml) was added the product of step (a) (20g) and diethyl

oxalate (29g) dissolved in ethanol (50ml). After refluxing for 15 hours the cooled solution was poured into a mixture of conc. HCl (5ml) and left for 10 minutes. The resulting mixture was evaporated and the residue was refluxed with methanol (40ml), water (100ml) and sodium bicarbonate (10g) for 1 hour. Methanol was evaporated and the aqueous residue was diluted with a little water, filtered and acidified to afford an oil. Repeated trituration with ether afforded the title compound (3.5g) mp 167-169°.

c) Sodium 6,8-diethyl-5-(2-hydroxypropoxy)-4-oxo-4H-1-benzopyran-2-carboxylate

The product of step b) (3.2g) and sodium bicarbonate (0.84g) were dissolved in water (100ml), filtered and the solution was lyophilised to afford the title compound (3.2g) mp > 300°.

Example 7

5-(2,3-Dihydroxy-n-propoxy)-4-oxo-8-n-propyl-4H-1-benzopyran-2-carboxylic acid

The sodium salt of 5-(2-hydroxy-n-propoxy)-4-oxo-8-n-propyl-4H-1-benzopyran-2-carboxylic acid was prepared as a 50% suspension in isotonic saline.

Animals

Male Cob Wistar rats (weight approximately 350g) were obtained from Charles River (Manston, Kent, UK).

Animal dosing and urine collection

Six rats were dosed twice daily with dosing solution (5ml) for 6 days. The daily dose was approximately 14g $kg^{-1}$ $day^{-1}$. The urine from each rat was collected daily and pooled.

Extraction of urine

The pooled urine was acidified to pH1 by the addition of concentrated HCl. The acidified urine was then extracted twice with two volumes of diethyl ether. The diethyl ether extract was evaporated to dryness under reduced pressure at $30^{o}$. The residue was redissolved in methanol (10ml).

High performance liquid chromatography

Aliquots (100 ul) of the extract or subsequently partially purified fractions were injected onto a Spherisorb 50DS 25 cm x 0.8mm (id) column. The column was eluted with a mobile phase consisting of 35% methanol 65% water containing ammonium acetate (5%) at a rate of 4ml $min^{-1}$ by pump. The column eluate was monitored at 350 nm by a UV detector.

Extraction

Combined fractions of eluate were concentrated under reduced pressure at $45^{o}$ to remove methanol. The concentrate was then acidified to pH 1 with concentrated HCl and extracted twice with two volumes of diethyl

ether. The ether was removed by evaporation under reduced pressure. The samples were then analysed and unchanged starting material together with the title compound were identified.

Example 8

5-(2,3-Dihydroxy-n-propoxy)-4-oxo-8-n-propyl-4H-1-benzopyran-2-carboxylic acid

a) Ethyl 4-oxo-5-(2-propenyloxy)-8-propyl-4H[1]benzopyran-2-carboxylate

A stirred solution of sodium metal, (47.1g), in dry ethanol, (1000ml), was treated dropwise with a solution of diethyl oxalate, (150.3g, 140ml), and 1-(2-hydroxy-6-(2-propenyloxy)-3-propylphenyl) ethanone, (95g), in dry ethanol, (300ml). The resulting mixture was heated at reflux for 3 hours, then left to cool overnight and poured into a mixture of conc. HCl (250ml), water, (1000ml), and chloroform, (1000ml). The organic layer was isolated, washed with water, (4 x 1000ml), dried ($MgSO_4$), filtered and evaporated to a red oil. This was heated under reflux for 1 hour in ethanol/C.HCl and the resulting solution was left overnight in a refrigerator. The resulting crystalline solid was filtered off and dried to constant weight, (68g). A portion (10g), was dissolved in chloroform and the solution was washed with $NaHCO_3$ solution, water, dried ($MgSO_4$), filtered and

evaporated. The residue was recrystallised from ethanol and dried *in vacuo* at 40° for 5 hours, leaving a yellow solid, (8.0g), mp 85-86°.

Elemental analysis found: C,68.5; H,6.4

$C_{18}H_{20}O_5$ Requires: C,68.34; H,6.37%

b) Ethyl 5-(2,3-Dihydroxy-n-propoxy)-4-oxo-8-n-propyl-4H-1-benzopyran-2-carboxylate

316Mg of the product of step a) (1mmol) in 5.5ml of a 63% solution of hydrogen peroxide in t-butanol (10ml) and 300 µl of a 0.5% solution of osmium tetroxide in t-butanol were stirred at room temperature for three hours. Thin layer chromatography monitoring in ether/acetone (9:1) revealed no further increase in product after three hours. The t-butanol was evaporated off under pressure and the residue taken up in a small volume of $CHCl_3$/$CH_3OH$ and applied to two preparative thin later chromatography plates which were developed in $CHCl_3$/$CH_3OH$ (9:1), the band due to the diol removed, and the silica eluted with $CHCl_3$/$CH_3OH$ (1:1). The organic solvent was removed under reduced pressure to give 262mg of slightly impure product which was recrystallised from aq. ethanol to give 163mg of the sub-title compound whose structure was confirmed by mass spectroscopy

c) 5-(2,3-Dihydroxy-n-propoxy)-4-oxo-8-n-propyl-4H-1-benzopyran-2-carboxylic acid

40Mg of the product of step b) (0.114mmol) and 9.6mg of $NaHCO_3$ (0.114 mmol) in 2mls of methanol were heated together at 70°. The reaction was monitored by thin layer chromatography in chloroform/methanol (3x10ml), acidified and extracted with $CHCl_3$/isopropanol (3x10mls). The organic solvent was removed under reduced pressure and trituration of the resulting gum with ether gave a yellow solid which was dried under vacuum to yield 12.6mg of the title compound whose structure was confirmed by NMR spectroscopy.

Example 9

5-(2,3-Dihydroxypropoxy)-4-oxo-8-propyl-4H[1] benzopyran-2-carboxylic acid

a) Ethyl 5-oxiranylmethoxy-4-oxo-8-propyl-4H[1]benzopyran -2-carboxylate

A solution of ethyl 4-oxo-5-(2-propenyloxy)-8-propyl -4H[1]benzopyran-2-carboxylate, (16.0g), in dry dichloromethane, (175ml), was stirred and cooled in an ice bath, then treated portionwise over 15 minutes with 3-chloroperoxybenzoic acid, (95% purity, 13.8g). The mixture was maintained at ice temperature for a further 2 hours, allowed to reach room temperature and then stirred for 90 hours. Excess oxidant was destroyed by addition of sodium bisulphite solution and the organic layer was washed with $NaHCO_3$ solution, water, and then dried and

evaporated. The residue was twice crushed under ether and filtered off, leaving the sub-title compound as a yellow solid (5.95g). The structure was confirmed by NMR spectroscopy.

b) Ethyl 5-(2,3-dihydroxypropoxy)-4-oxo-8-propyl-4H[1] benzopyran-2-carboxylate

A solution of the epoxide from step a), (3g), was dissolved in acetone, (100ml), and treated at room temperature with 3M $H_2SO_4$, (15ml). The resulting solution was left to stand for 1.5 hours and then evaporated to dryness. The residue was diluted with water and extracted into ethyl acetate. The extracts were washed with water, brine, water again, then dried, ($MgSO_4$), filtered and evaporated. The residue, (1.8g), was chromatographed on silica gel, (90g, 70-230 mesh), eluting in order with $CH_2Cl_2$:ethylacetate 1:1, neat ethyl acetate, and ethyl acetate:ethanol 97:3, which gave the required product. The fractions were evaporated and the residue was triturated with ether, leaving a yellow solid, (1.0g) mp 141-142$^o$.

Elemental analysis found: C,62.08; H,6.45

$C_{18}H_{22}O_7$ requires: C,61.7; H,6.3%

c) 5-(2,3-Dihydroxypropoxy)-4-oxo-8-propyl-4H[1] benzopyran-2-carboxylic acid

A solution of the ethyl ester from step b) (0.87g),

in ethanol, (60ml), was treated dropwise at reflux with 2M NaOH solution, (1.25ml). After 10 minutes the mixture was evaporated and the residue was dissolved in water. This solution was washed with ether, warmed under vacuum and then filtered. The filtrate was acidified and the precipitate was extracted into ethyl acetate. The extracts were dried, ($MgSO_4$), filtered and evaporated, the residue was crushed under ether and filtered off, leaving the sub-title compound as a pale yellow solid, (0.65g) mp 143-145$^{\circ}$.

d) Sodium 5-(2,3-dihydroxypropoxy)-4-oxo-8-propyl-4H[1] benzopyran-2-carboxylate

A solution of the carboxylic acid from step c) (0.49g), in ethanol, (25ml), was treated dropwise at room temperature with 0.5M NaOH solution, (3.04ml). The solution was evaporated and the residue was dissolved in sterile water, filtered and the filtrate freeze-dried overnight, giving the required salt as a yellow solid (0.49g).

Analytical HPLC reports 99.8% purity.

Thermal gravimetric analysis showed 5.5% wt. loss at 150$^{\circ}$.

Elemental analysis found: C,52.42; H,5.29

$C_{16}H_{17}NaO_7$ 5.5% $H_2O$ requires: C,52.73; H,5.33%

Example 10

5-(2-Hydroxypropoxy)-4-oxo-8-phenylmethyl-4H-1-benzopyran-2-carboxylic acid

a) 1-(2,6-Dihydroxy-3-phenylmethylphenyl)ethanone

2,6-Dihydroxy acetophenone (38g), chloroform (500ml), 60/80 petroleum ether (500ml) and orthophosphoric acid (200g) were refluxed and stirred while a solution of benzyl alcohol (53.25g) in chloroform (100ml) was added over 1 hour. When addition was complete refluxing was continued for 3 hours. The mixture was then cooled and the organic phase was decanted, washed with water and dried. Evaporation afforded a gum which was triturated with petroleum ether and recrystallised from $CCl_4$ to afford the sub-title compound 39.5g mp 117-119°.

b) 1-(2-Hydroxy-6-[2-hydroxypropoxy]-3-phenylmethylphenyl) ethanone

The product of step a) (18.1g), propylene oxide 6.5g, benzyltrimethylammonium hydroxide (0.75ml) and dioxan (75ml) were heated in a sealed container at 100° overnight. After cooling, volatile materials were removed under vacuum and the residue was dissolved in ether, washed with 1N NaOH, water, dil. HCl and water. Drying and evaporation afforded the sub-title compound 9.9g. NMR spectrum consistent with the desired structure.

c) 5-(2-Hydroxypropoxy)-4-oxo-8-phenylmethyl-4H-1-benzopyran-2-carboxylic acid

The product of step b) (9.6g) was added to a solution of sodium (3.68g) in ethanol (500ml), and then diethyl oxalate (11.84g) was added. The mixture was refluxed for 1.25 hours, then poured into $CHCl_3$ (800ml) and dil. HCl (100ml). The organic phase was evaporated, and the residue was treated with conc. $H_2SO_4$ (1ml) in ethanol (50ml) at room temperature for 15 minutes. Ethanol was evaporated and the oil was dissolved in 1:1 ethanol-water (150ml) and treated with sodium bicarbonate (30gm). This mixture was refluxed for 1 hour and then the ethanol was evaporated. The aqueous solution was washed with ethyl acetate, acidified and extracted into $CHCl_3$. Evaporation gave a gum which was triturated with ether, and recrystallised from ethyl acetate to afford the sub-title compound (1.2g) mp 170-2$^{o}$.

d) <u>Sodium 5-(2-hydroxypropoxy)-4-oxo-8-phenylmethyl-4H-1-benzopyran-2-carboxylate</u>

The product of step c) (3.54g) and sodium bicarbonate (0.84g) were heated together in water (100ml) until dissolved. The solution was filtered and the solution lyophilised to afford the sub-title compound (3.6g) mp > 300$^{o}$.

<u>Example 11</u>

<u>5-(2-Benzoyloxypropoxy)-4-oxo-8-propyl-4H-1-benzopyran-2-carboxylic acid</u>

a) Ethyl 5-(2-benzoyloxypropoxy)-4-oxo-8-propyl-4H-1-benzopyran-2-carboxylate

A mixture of ethyl 5-(2-hydroxypropoxy)-4-oxo-8-propyl-4H-1-benzopyran-2-carboxylate (5g), dry pyridine, (6ml) and benzoyl chloride, (4g) in dry 1,2-dichloroethane, (50ml) was heated under reflux for 1 hour, and then left at ambient temperature overnight. Volatile components were then evaporated and the residue was triturated with cold dilute hydrochloric acid, washed with water, triturated with cold sodium bicarbonate solution, washed with water and then dried, *in vacuo* at 50°, to leave the sub-title material as a tacky, pale orange oil, (5.6g). The proposed structure was supported by NMR studies and by mass spectroscopy.

b) 5-(2-Benzoyloxypropoxy)-4-oxo-8-propyl-4H-1-benzopyran-2-carboxylic acid

A solution of the product of step a) (5.5g) in dimethylformamide, (25ml) was heated on a steam bath and treated over 15 min with a solution of sodium bicarbonate (3g) in water, (40ml). This mixture was heated on a steam bath for a further 1.25 hour, evaporated to low bulk (25g) cooled and diluted with water. The resulting mixture was filtered and the filtrate was carefully acidified with dilute hydrochloric acid. A solid was precipitated, which was filtered off, washed with water, and then dissolved in

ether. The ethereal solution was dried ($Na_2SO_4$), filtered and then diluted with 40-60° petroleum ether. Crystallization occurred and gave the sub-title material as a pale cream powder (1.95g), mp 108-10°. Satisfactory nuclear magnetic resonance and mass spectroscopic data were obtained.

c) Sodium 5-(2-benzoyloxypropoxy)-4-oxo-8-propyl-4H-1-benzopyran-2-carboxylate

A mixture of the product of step b) (1.85g) and sodium bicarbonate, (0.4g) in water (8ml) was stirred to give a clear solution, which was flooded with acetone. The precipitated solid was filtered off and discarded and the filtrate was evaporated to dryness. The residue was re-dissolved in dry acetone and the solution was diluted with dry ether. Crystallization occurred and gave sub-title material as an off-white powder (1.6g). The proposed structure was supported by nuclear magnetic resonance and mass spectroscopic data.

Analysis: Found: C,62.92; H,4.80

$C_{23}H_{21}NaO_7$ 1.5%$H_2O$ Requires: C,62.9; H,4,78-4.92

Example 12

Butanedioic acid, mono(2-ethoxycarbonyl-4-oxo-8-propyl-4H-1-benzopyran-5-yloxy)prop-2-yl ester

a) Butanedioic acid mono(2-ethoxycarbonyl-4-oxo-8-propyl-4H-1-benzopyran-5-yloxy)prop-2-yl ester

A solution of ethyl 5-(2-hydroxypropoxy)-4-oxo-8-propyl-4H-1-benzopyran-2-carboxylate, (9.8g) in pyridine (100ml) was treated with succinic anhydride, (5g) and the resulting solution was heated on a steam bath for 17hr. Most of the pyridine was evaporated off and the concentrate was exhaustively triturated with cold N HCl, then with water and dried to leave sub-title material as an off-white powder, (13g), m.p. 146-9$^{o}$. Satisfactory N.M.R. spectra were obtained.

b) Butanedioic acid, mono(2-ethoxycarbonyl-4-oxo-8-propyl-4H-1-benzopyran-5-yloxy)prop-2-yl ester mono sodium salt

The product of step a) (1.5g) was carefully dissolved in a solution of sodium bicarbonate, (0.3g) in water (15ml). The solution was filtered and freeze-dried to leave the sub-title material as a straw coloured, deliquescent solid, (1.2g).

Analysis:

Found: C, 55.37; H, 7.22

$C_{22}H_{25}NaO_9$ 1.25$H_2O$ requires: C,55.17,H, 5.53%

Example 13

5-(2-Hydroxypropoxy)-4-oxo-8-propyl-4H-1-thiabenzopyran -2-carboxylic acid

a) Ethyl 4-oxo-5-propargyloxy-8-propyl-4H-1-thiabenzopyran -2-carboxylate.

A mixture of 80% w/w propargyl bromide in toluene (20g) and ethyl 5-hydroxy-4-oxo-8-propyl-4H-thiabenzopyran-2-carboxylate, (14.6g) in dry N,N-dimethylformamide, (75ml) was stirred and cautiously treated with 50% w/w sodium hydride in oil. The mixture was heated on a steam bath for 18hr. Volatile materials were removed and the residue was extracted with ether. This extract was filtered, evaporated, and the residue was extracted with boiling 40-60° petroleum ether. Material remaining undissolved was subsequently crytallised from a 60-80° petroleum ether/ethyl acetate mixture to give sub-title material as brown crystals, (5g), m.p. 95-8°.

a) <u>Ethyl 5-acetonyloxy-4-oxo-8-propyl-4H-1-thiabenzopyran -2-carboxylate</u>

A mixture of the product of step a) (4.4g), with 40% boron trifluoride: acetic acid complex, (0.3g) and red mercuric oxide, (0.04g) in glacial acetic acid, (40ml), was heated on a steam bath for 1 hr. Most of the solvent was evaporated off and the contentrate was diluted with water. The aqueous phase was discarded and the organic deposit was extracted into ethyl acetate. This extract was diluted with 60-80° petroleum ether and filtered. The filtrate, upon further dilution with 60-80° petroleum ether, deposited an oil, which upon standing solidified to leave the sub-title material as a brown

powder, (3.25g) m.p. 88-98°.

c) Ethyl 5-(2-hydroxypropoxy)-4-oxo-8-propyl-4H-1-thiabenzopyran-2-carboxylate

A solution of the product of step b) (2.15g) and glacial acetic acid, (0.8g) in ethanol, (40ml) was stirred and treated over a few minutes with sodium borohydride, (0.4g). Stirring was continued for 10 min. and the mixture was then allowed to stand for 10 min. Volatile materials were removed and the residue was partitioned between ether and water. The ethereal layer was isolated and evaporated and the residue was crystallised, from an ethyl acetate/60-80° petroleum ether mixture, to give the sub-title material as orange crystals, (1.2g), m.p. 102-7°.

d) Sodium 5-(2-hydroxypropoxy)-4-oxo-8-propyl-1-thiabenzopyran-2-carboxylate

A mixture of the product of step c) (1.2g), sodium bicarbonate, (0.6g), water, (20ml) and methanol, (20ml) was heated under reflux for 40 min. and then most of the solvent was removed. The concentrate was diluted with water, and then evaporated to low bulk. This concentrate was diluted with acetone and excess inorganic material was filtered off. The filtrate was evaporated to near dryness and then treated with anhydrous acetone. Crystallization

occurred yielding the sub-title material as a fawn powder, (0.86g)

Analysis:

Found: C, 53.56; H, 5.21

$C_{16}H_{17}NaO_5S.0.75H_2O$ requires: C,53.7,H, 5.17%

Example 14

8-(1,1-Dimethylethyl)-5-(2-hydroxypropoxy)-4-oxo-4H[1]benzopyran-2-carboxylic acid

a) 2,6-Dihydroxy-3-(1,1-dimethylethyl)phenylethanone

A stirred mixture of 2,6-dihydroxyphenylethanone, (30.4g), orthophosphoric acid, (160g), chloroform, (400ml), and petroleum ether, (400ml, bp 60-80$^o$) was heated under reflux and treated dropwise over 1.25h with a solution of t-butanol, (29.6ml), in chloroform, (80ml). The resulting mixture was heated under reflux for a further 4h, evaporated to lower bulk, (250ml), and cooled to room temperature. Chloroform (1.5 l), was added and the supernatant yellow solution was decanted from $H_3PO_4$. The organic solution was washed with brine, dried, ($MgSO_4$), filtered and evaporated to near dryness. Petroleum ether, (1.5 l bp40-60$^o$) was added, the solid was filtered off, washed with petroleum ether and dried, giving the required material as a yellow crystalline solid, (23g). Recrystallisation from ether/petroleum ether bp40-60$^o$ gave the sub-title

compound, mp 187-9°. Structure was confirmed by NMR.

b) 3-(1,1-Dimethylethyl)-2-hydroxy-6-(2-hydroxypropoxy) phenylethanone

A solution of the product from step (a), (31.5g), propylene oxide, (13.05g) and benzyltrimethyl ammonium hydroxide, (2.5ml of a 40% solution), in 1,4-dioxan (125ml), was heated to 100° in a sealed vessel for 16hr. The solvent was removed and the residue was dissolved in ether. This solution was washed with 1M NaOH solution, water, dil. HCl, water again, dried, ($MgSO_4$), filtered and evaporated to dryness, leaving the sub-title compound as a green oil, (36.0g).

c) Ethyl 8-(1,1-dimethylethyl)-5-(2-hydroxypropoxy)-4-oxo-4H[1]benzopyran-2-carboxylate

A stirred solution of sodium metal, (15.5g), in ethanol, (500ml), was treated with the product from step (b), (36.0g) and then with diethyl oxalate, (49.0g). This mixture was heated at reflux for 45 min., cooled and poured into chloroform, (1500ml), containing excess conc. HCl and water. The organic layer was isolated, evaporated, and the residue was heated under reflux with ethanol, (250ml) and conc. HCl, (10 drops), for 45min. The ethanol was evaporated off and the residue was triturated with ether. Insoluble material was isolated by filtration and dried *in vacuo*, leaving a pale yellow

solid, (23.9g). This solid was recrystallised from benzene/petroleum ether to give material, mp 110-111°.

Analysis found: C, 65.2; H,7.1

$C_{19}H_{24}O_6$ required: C, 65.5; H,6.94%

d) <u>8-(1,1-Dimethylethyl)-5-(2-hydroxypropoxy)-4-oxo-4H [1]benzopyran-2-carboxylic acid</u>

A mixture of the ester from step (c), (17.6g), in methanol, (100ml), was treated with a solution of $NaHCO_3$,(17.6g), in water, (500ml). The resulting cloudy mixture was heated under reflux for 1.5h and then the methanol was evaporated off. The aqueous solution was filtered and acidified to pH 1 with conc. HCl. After standing the supernatant solution was decanted off and the residual gum was rinsed with clean water and then triturated with petroleum ether. The resulting solid was filtered off, dried, (15g), and crystallised from aqueous acetone to give the required acid, (14.0g), mp 93-95°.

Analysis found:- C, 63.45; H, 6.45

$C_{17}H_{20}O_6$ requires:- C, 63.74; H, 6.29%

e) <u>Sodium 8-(1,1-dimethylethyl)-5-(2-hydroxypropoxy) -4-oxo-4H[1]benzopyran-2-carboxylate</u>

The acid from step (d), (10.7g), was added to a solution of $NaHCO_3$, (2.73g), in water (50ml). The resulting solution was filtered, freeze dried and the product was dried *in vacuo*, (10.4g).

Analysis found: C, 57.6; H, 5.8

$C_{17}H_{19}NaO_6$.3.4% water requires: C, 57.6; H, 5.8%.

Example 15

8-(2-Hydroxypropoxy)-9-oxo-5-propyl-1H,9H-benzopyrano [2,3-d]-v-triazole

a) 9-Oxo-8-(2-oxopropoxy)-5-propyl-1H,9H-benzopyrano [2,3-d]-v-triazole

8-Hydroxy-9-oxo-5-propyl-1H,9H-benzopyrano[2,3-d]-v-triazole (24.5g), potassium carbonate (27.6g), dry dimethylformamide (500ml) and chloroacetone (9.2g) were stirred together for 4 days at room termpature under nitrogen. The mixture was diluted with water (5L) and the pH was adjusted to 7 with acetic acid. The aqueous solution was extracted with ethyl acetate which was then evaporated. The residue was separated by high performance liquid chromatography to afford the sub-title compound (0.2g). Structure was supported by m.s.

b) Sodium salt of 8-(2-hydroxypropoxy)-9-oxo-5-propyl-1H,9H-benzopyrano[2,3-d]-v-triazole

The product of step a) was treated with sodium borohydride (0.1g) in ethanol (20ml) at room termperature for 24hr, then the mixture was acidified with acetic acid and evaporated. The residue was extracted well with ethyl acetate, which was evaporated to afford the hydroxy compound (0.05g). This gummy solid was treated with

sodium hydroxide (1.6ml of 0.1N solution) at room temperature. After stirring for 30 min the solvent was removed by lyophilisation to afford the title compound (0.58g) mp $>$ 300°C.

What we claim is:-

1. A compound of formula I,

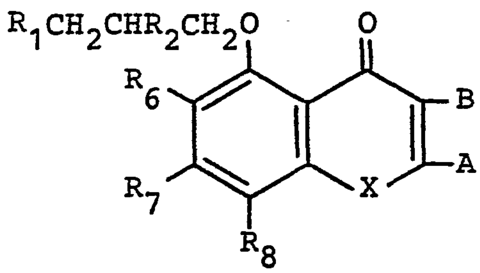

I

in which X is -O-, -S- or $-NR_3-$,

$R_3$ is hydrogen or alkyl C 1 to 6,

A is -COOH or 5-(1H-tetrazolyl), and B is hydrogen, or A and B together form a chain -N=N-NH-,

$R_1$ is hydrogen or hydroxy,

$R_2$ is hydroxy or esterified hydroxy,

$R_6$ is hydrogen or alkyl C 1 to 3,

$R_7$ is hydrogen or methyl, and

$R_8$ is alkyl C 1 to 4 optionally substituted by phenyl,

provided that

when B is hydrogen, A is -COOH, X is -O-, $R_1$ is hydrogen, $R_2$ is hydroxy, $R_6$ and $R_7$ are both hydrogen then $R_8$ is not propyl,

and pharmaceutically acceptable derivatives thereof.

2. A compound according to Claim 1, wherein B is

hydrogen and A is -COOH.

3. A compound according to Claim 1 or 2, wherein X is -S- or -O-.

4. A compound according to any one of the preceding claims, wherein $R_1$ is hydrogen.

5. A compound according to any one of the preceding claims, wherein $R_2$ is hydroxy esterified by a C 1 to 7 hindered monocarboxylic acid, with an aryl C7 to 12 acid, or with a C3 to 7 dibasic acid.

6. A compound according to Claim 5, wherein $R_2$ is hydroxy esterified by benzoic acid.

7. A compound according to any one of the preceding claims, wherein $R_6$ is hydrogen, ethyl or propyl, $R_7$ is hydrogen and $R_8$ is ethyl, propyl, butyl or benzyl.

8. 5-[5-(2-Hydroxypropoxy)-4-oxo-8-propyl-4H-1-benzopyran-2-yl]1H-tetrazole,

1-Ethyl-1,4-dihydro-5-(2-hydroxypropoxy)-4-oxo-8-propylquinoline-2-carboxylic acid,

1,4-Dihydro-5-(2-hydroxypropoxy)-4-oxo-8-propylquinoline-2-carboxylic acid,

5-(2-Hydroxypropoxy)-4-oxo-6,8-dipropyl-4H-1-benzopyran-2-carboxylic acid,

5-(2-Hydroxypropoxy)-7-methyl-4-oxo-8-propyl-4H-1-benzopyran-2-carboxylic acid,

6,8-Diethyl-5-(2-hydroxypropoxy)-4-oxo-4H-1-benzopyran-2-carboxylic acid,

5-(2,3-Dihydroxy-n-propoxy)-4-oxo-8-n-propyl-4H-1-benzopyran-2-carboxylic acid,

5-(2-Hydroxypropoxy)-4-oxo-8-phenylmethyl-4H-1-benzopyran-2-carboxylic acid,

5-(2-Benzoyloxypropoxy)-4-oxo-8-propyl-4H-1-benzopyran-2-carboxylic acid,

Butanedioic acid, mono(2-ethoxycarbonyl-4-oxo-8-propyl-4H-1-benzopyran-5-yloxy)prop-2-yl ester,

5-(2-Hydroxypropoxy)-4-oxo-8-propyl-4H-1-thiabenzopyran-2-carboxylic acid,

8-(1,1-Dimethylethyl)-5-(2-hydroxypropoxy)-4-oxo-4H[1]benzopyran-2-carboxylic acid,

8-(2-Hydroxypropoxy)-9-oxo-5-propyl-1H,9H-benzopyrano[2,3-d]-v-triazole,

or the sodium salt of any one thereof.

9. A pharmaceutical composition conprising a compound according to any one of the preceding claims and a pharmaceutically acceptable adjuvant, diluent or carrier.

10. A process for the production of a compound of formula I, or a pharmaceutically acceptable derivative thereof, which comprises

a) producing a compound of formula I in which A and B together form a chain -N=N-NH- by removing a protecting group L from a compound of formula III,

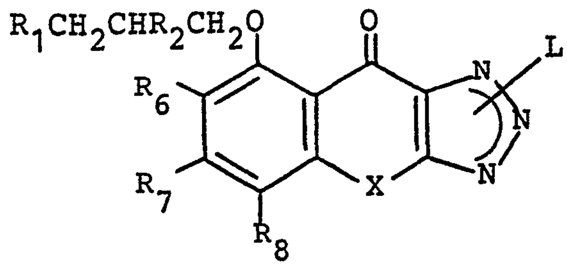

III

in which X, $R_1$, $R_2$, $R_6$, $R_7$ and $R_8$ are as defined above, and

L is a protecting group,

b) producing a compound of formula I in which B is hydrogen and A is 5-(1H-tetrazolyl), by reacting a compound of formula IV,

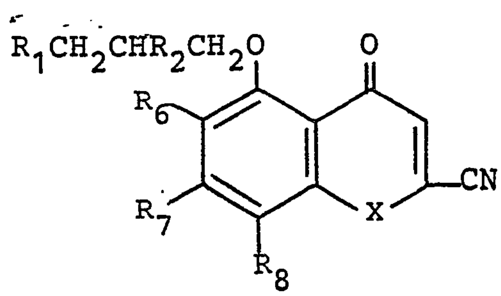

IV

in which X, $R_1$, $R_2$, $R_6$, $R_7$ and $R_8$ are as defined above,

with an azide, in solvent which is inert under the reaction conditions,

c) producing a compound of formula I in which $R_1$ or $R_2$ is hydroxy by selectively reducing a compound of formula V,

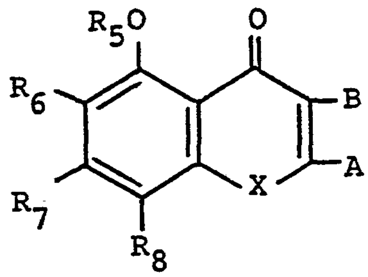

V

or a salt, ester or amide thereof,

in which X, A, B, $R_6$, $R_7$ and $R_8$ are as defined above, and

$R_5$ represents a group $-CH_2CZCHZ$, where one Z represents carbonyl oxygen and the other represents $H_2$,

with a suitable selective carbonyl reducing agent,

d) producing a compound of formula I in which B is hydrogen and A is -COOH by hydrolysing a compound of formula VI,

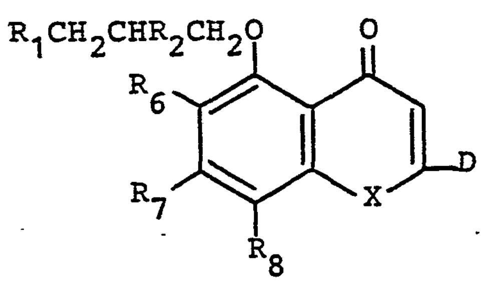

VI

in which D represents a group hydrolyseable to -COOH, and

X, $R_1$, $R_2$, $R_6$, $R_7$ and $R_8$ are as defined above,

e) producing a compound of formula I in which B is hydrogen and A is -COOH by cyclising a compound of formula VII,

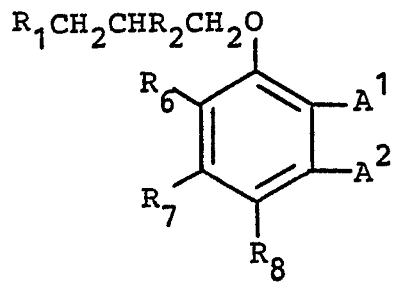

VII

in which $R_1$, $R_2$, $R_6$, $R_7$ and $R_8$ are as defined above,

$A^1$ and $A^2$ represent the pairs of groups

i) $-COCH_2COCOR''$ and -OM, or

ii) -H and -X-C(COR")=CH-COR"

X is as defined above,

R" represents -OM, or a group which is hydrolysable thereto, and

M represents hydrogen or a cation,

and if necessary or desired hydrolysing the group -COR", to a group -COOM,

f) reacting a compound of formula VIII,

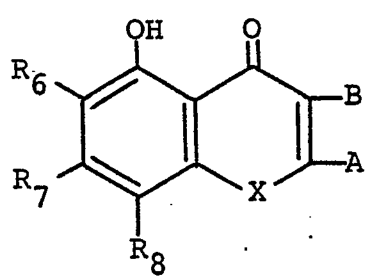

VIII

or a salt, ester or amide thereof,

in which X, A, B, $R_6$, $R_7$ and $R_8$ are as defined above,

with propylene oxide or a compound of formula IX,

$$R_1CH_2CHR_2CH_2G \qquad IX$$

in which G is a good leaving group,

g) producing a compound of formula I in which $R_1$ and $R_2$ are both hydroxy by

i) hydrolysing a compound of formula X,

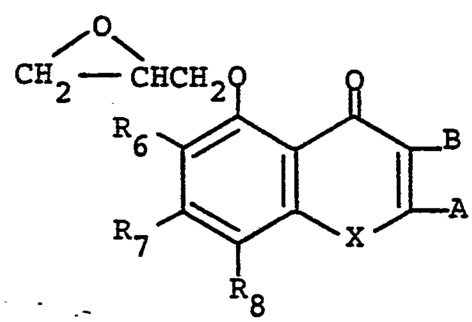

X

or a salt, ester or amide thereof,

in which X, A, B, $R_6$, $R_7$ and $R_8$ are as defined above, or

ii) subjecting a compound of formula XI,

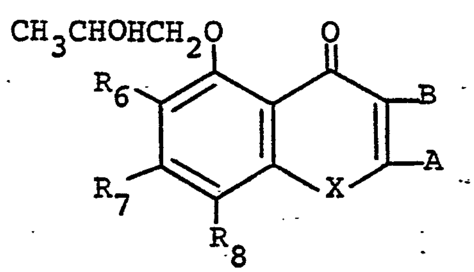

XI

in which X, A, B, $R_6$, $R_7$ and $R_8$ are as defined above,

or a salt, ester or amide thereof,

to mammalian metabolism, or

iii) reaction of a compound of formula XII,

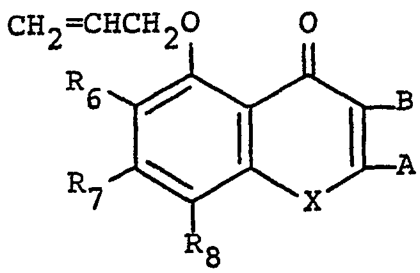

XII

or a salt, ester or amide thereof,

in which X, A, B, $R_6$, $R_7$ and $R_8$ are as defined above,

with osmium tetroxide, and hydrolysis of the resulting complex, or

h) producing a compound of formula I in which $R_2$ is esterified hydroxy, by reaction of a corresponding compound of formula I, or a salt, carboxylic ester or amide thereof, in which $R_2$ is hydroxy with an appropriate acid, or a derivative thereof,

and where desired or necessary converting the resulting compound of formula I to a pharmaceutically acceptable derivative thereof, or vice versa.

11. A compound according to any one of Claims 1 to 9 for use as a pharmaceutical.

What we claim is:-

1. A process for the production of a compound of formula I,

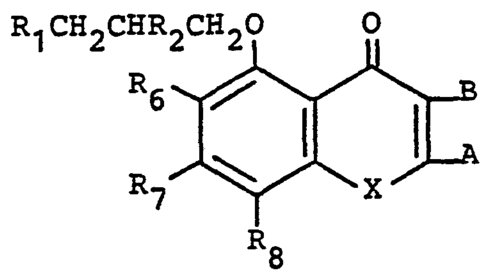

I

in which X is -O-, -S- or $-NR_3-$,

$R_3$ is hydrogen or alkyl C 1 to 6,

A is -COOH or 5-(1H-tetrazolyl), and B is hydrogen, or A and B together form a chain -N=N-NH-,

$R_1$ is hydrogen or hydroxy,

$R_2$ is hydroxy or esterified hydroxy,

$R_6$ is hydrogen or alkyl C 1 to 3,

$R_7$ is hydrogen or methyl, and

$R_8$ is alkyl C 1 to 4 optionally substituted by phenyl,

provided that

when B is hydrogen, A is -COOH, X is -O-, $R_1$ is hydrogen, $R_2$ is hydroxy, $R_6$ and $R_7$ are both hydrogen then $R_8$ is not propyl,

or a pharmaceutically acceptable derivative thereof,

which comprises

a) producing a compound of formula I in which A and B together form a chain -N=N-NH- by removing a protecting group L from a compound of formula III,

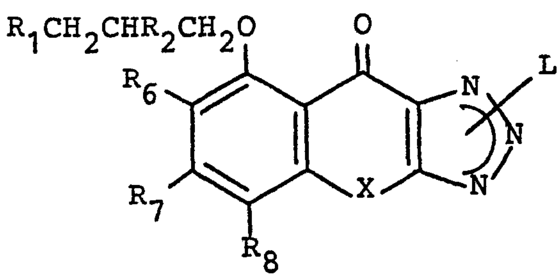

III

in which X, $R_1$, $R_2$, $R_6$, $R_7$ and $R_8$ are as defined above, and

L is a protecting group,

b) producing a compound of formula I in which B is hydrogen and A is 5-(1H-tetrazolyl), by reacting a compound of formula IV,

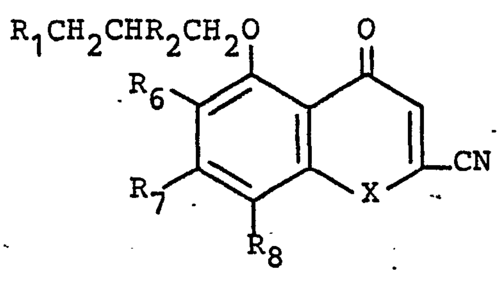

IV

in which X, $R_1$, $R_2$, $R_6$, $R_7$ and $R_8$ are as defined above,

with an azide, in solvent which is inert under the reaction conditions,

c) producing a compound of formula I in which $R_1$ or $R_2$ is hydroxy by selectively reducing a compound of formula V,

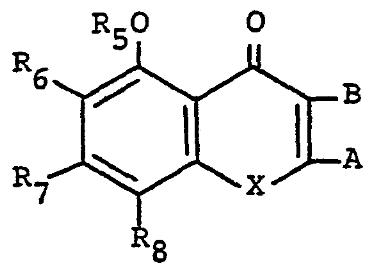

V

or a salt, ester or amide thereof,

in which X, A, B, $R_6$, $R_7$ and $R_8$ are as defined above, and

$R_5$ represents a group $-CH_2CZCHZ$, where one Z represents carbonyl oxygen and the other represents $H_2$,

with a suitable selective carbonyl reducing agent,

d) producing a compound of formula I in which B is hydrogen and A is -COOH by hydrolysing a compound of formula VI,

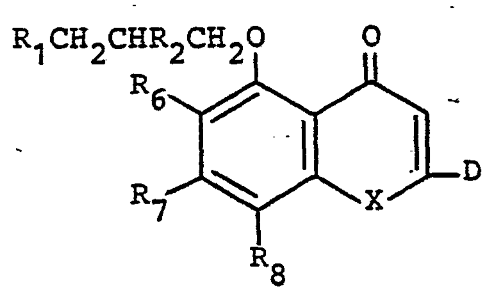

VI

in which D represents a group hydrolyseable to -COOH, and

X, $R_1$, $R_2$, $R_6$, $R_7$ and $R_8$ are as defined above,

e) producing a compound of formula I in which B is hydrogen and A is -COOH by cyclising a compound of formula VII,

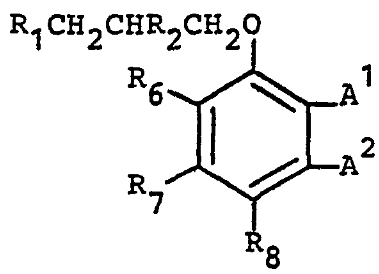

VII

in which $R_1$, $R_2$, $R_6$, $R_7$ and $R_8$ are as defined above,

$A^1$ and $A^2$ represent the pairs of groups

i) $-COCH_2COCOR"$ and -OM, or

ii) -H and -X-C(COR")=CH-COR"

X is as defined above,

R" represents -OM, or a group which is hydrolysable thereto, and

M represents hydrogen or a cation,

and if necessary or desired hydrolysing the group -COR", to a group -COOM,

f) reacting a compound of formula VIII,

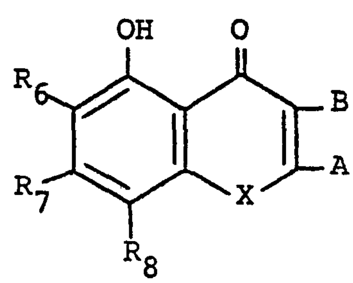

VIII

or a salt, ester or amide thereof,

in which X, A, B, $R_6$, $R_7$ and $R_8$ are as defined above,

with propylene oxide or a compound of formula IX,

$$R_1CH_2CHR_2CH_2G \qquad IX$$

in which G is a good leaving group,

g) producing a compound of formula I in which $R_1$ and $R_2$ are both hydroxy by

i) hydrolysing a compound of formula X,

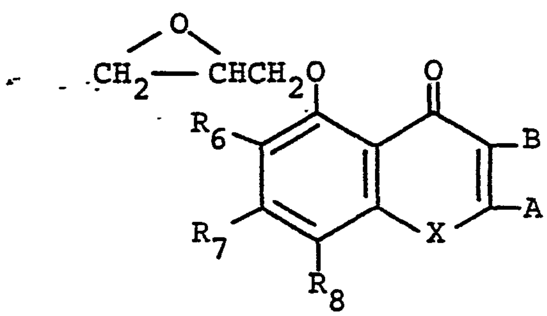

X

or a salt, ester or amide thereof,

in which X, A, B, $R_6$, $R_7$ and $R_8$ are as defined above, or

ii) subjecting a compound of formula XI,

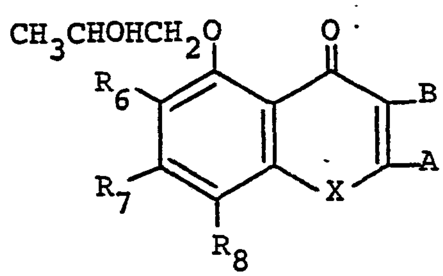

XI

in which X, A, B, $R_6$, $R_7$ and $R_8$ are as defined above,

or a salt, ester or amide thereof,

to mammalian metabolism, or

iii) reaction of a compound of formula XII,

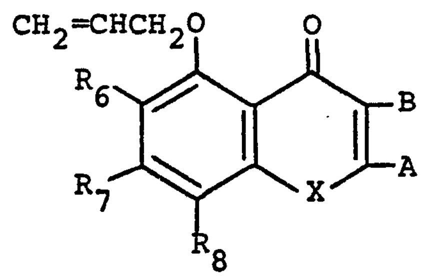

XII

or a salt, ester or amide thereof,

in which X, A, B, $R_6$, $R_7$ and $R_8$ are as defined above,

with osmium tetroxide, and hydrolysis of the resulting complex, or

h) producing a compound of formula I in which $R_2$ is esterified hydroxy, by reaction of a corresponding compound of formula I, or a salt, carboxylic ester or amide thereof, in which $R_2$ is hydroxy with an appropriate acid, or a derivative thereof,

and where desired or necessary converting the resulting compound of formula I to a pharmaceutically acceptable derivative thereof, or vice versa.

2. A process according to Claim 1, wherein B is

hydrogen and A is -COOH.

3. A process according to Claim 1 or 2, wherein X is -S- or -O-.

4. A process according to any one of the preceding claims, wherein $R_1$ is hydrogen.

5. A process according to any one of the preceding claims, wherein $R_2$ is hydroxy esterified by a C 1 to 7 hindered monocarboxylic acid, with an aryl C7 to 12 acid, or with a C3 to 7 dibasic acid.

6. A process according to Claim 5, wherein $R_2$ is hydroxy esterified by benzoic acid.

7. A process according to any one of the preceding claims, wherein $R_6$ is hydrogen, ethyl or propyl, $R_7$ is hydrogen and $R_8$ is ethyl, propyl, butyl or benzyl.

8. A process according to Claim 1, wherein the compound of formula I is

5-[5-(2-Hydroxypropoxy)-4-oxo-8-propyl-4H-1-benzopyran-2-yl]1H-tetrazole,

1-Ethyl-1,4-dihydro-5-(2-hydroxypropoxy)-4-oxo-8-propylquinoline-2-carboxylic acid,

1,4-Dihydro-5-(2-hydroxypropoxy)-4-oxo-8-propylquinoline-2-carboxylic acid,

5-(2-Hydroxypropoxy)-4-oxo-6,8-dipropyl-4H-1-benzopyran-2-carboxylic acid,

5-(2-Hydroxypropoxy)-7-methyl-4-oxo-8-propyl-4H-1-

benzopyran-2-carboxylic acid,

6,8-Diethyl-5-(2-hydroxypropoxy)-4-oxo-4H-1-benzopyran-2-carboxylic acid,

5-(2,3-Dihydroxy-n-propoxy)-4-oxo-8-n-propyl-4H-1-benzopyran-2-carboxylic acid,

5-(2-Hydroxypropoxy)-4-oxo-8-phenylmethyl-4H-1-benzopyran-2-carboxylic acid,

5-(2-Benzoyloxypropoxy)-4-oxo-8-propyl-4H-1-benzopyran-2-carboxylic acid,

Butanedioic acid, mono(2-ethoxycarbonyl-4-oxo-8-propyl-4H-1-benzopyran-5-yloxy)prop-2-yl ester,

5-(2-Hydroxypropoxy)-4-oxo-8-propyl-4H-1-thiabenzopyran-2-carboxylic acid,

8-(1,1-Dimethylethyl)-5-(2-hydroxypropoxy)-4-oxo-4H[1]benzopyran-2-carboxylic acid,

8-(2-Hydroxypropoxy)-9-oxo-5-propyl-1H,9H-benzopyrano[2,3-d]-v-triazole,

or the sodium salt of any one thereof.